# EUROPEAN PATENT APPLICATION

(11) **EP 0 748 639 A2**
(43) Date of publication of application: **18.12.1996**
(21) Application number: 96500076.3
(22) Date of filing: 14.06.1996
(51) Int. Cl.: A61N 5/06

(54) **Selective chromotherapy self application device**

(30) Priority: 14.06.1995 ES 9501193
(71) Applicant: Julia Fernandez, Rafael, 20750 Zumaia (Guipuzcoa) (ES)
(72) Inventor: Julia Fernandez, Rafael, 20750 Zumaia (Guipuzcoa) (ES)
(74) Representative: Perez Bonal, Bernardo

(57) **Abstract**

Is based on the vibratory activation of a coloured light source whenever that light goes through a natural quartz crystal containing traces of foreign matter and transparency imperfections and includes a crystalline translucent supporting element 1, organic or mineral, mass dyed and which acts as a support of one or more disperse layers of natural mineral quartz 2, crushed down to a grain size distribution ranging from impalpable dust to three millimetres, being this crushed matter fully or partially adhered to its supporting element 1 and arranged inside a closed chamber 7 or arranging both sets onto positioning templates 8 of groups of different or similar shape and colour, that place each such set over the required area of the body.

## Description

### OBJECT OF THE INVENTION

The object of the invention is a selective chromotherapy self application device that makes it possible to apply the various variants of this therapeutic technique at the home of the patient, given that it is the patient himself who selects the proper place and time to put into practise the treatment prescribed by the medical specialist as set out in a text specific to the diagnosed disease.

To that end the user acquires means that are located at previously specified regions of his body and subjects same to the action of light source with a colour temperature of not less than 3200 degrees Kelvin, as per previously mentioned scale. Halogen and tungsten lamps are therefore valid for this purpose, although this is not the case with over-voltaged lamps because of their extremely short operating life and the very cold light, above 5500 degrees K, that they give off.

### BACKGROUND OF THE INVENTION

Chromotherapy is an ailing science using colours starting off undeniable scientific facts such as, for instance, that light is life, but that without the presence of light no biological phenomena may prosper at a normal scale.

Chromotherapy is the science that uses different colours to change or maintain body vibrations at the frequency that brings about calm and harmony. These colour beams may be visible or invisible to the human eye and may be applied to the body either physically, by way of a certain exposure to the light beams themselves, or mentally, using suggestion, visualization or meditation techniques.

Healing using colours was, probably, the very first type of therapy used by mankind, as it was the method supplied by nature as a natural way to keep the human organism properly balanced and regulated. When walked on earth for the very first time the rays of the sun fed him and kept him warm, flora and fauna colours affected his state of mind and temperament, the rhythm of the winds and the whisper of the sea cradled his sleep. It may well be that he did not have the technical knowledge and skills of modern man, but neither did he suffer the psychogenic and iatrogenic diseases induced by medical suggestions and practices. Why? Because primitive man had sufficient wisdom to live according to the laws of nature. Colours are basic for any therapeutic system.

According to chromotherapy science there are seven basic postulates as follows:
1. All objects have characteristic vibration frequencies.
2. All organs have characteristic vibration frequencies, under healthy conditions.
3. Disease is an altered function that constitutes the natural response of the body to pressure. The altered function is nothing other than a frequency change, the increase or decrease of vibrations caused by a chemical, mechanical or thermal stressing factor. Germs are but one among many hundreds of stressing factors. Thus all illnesses have characteristic vibration frequencies.
4. The application of the proper frequency, be it in the form of food, medicine, drugs, etc. shall influence the altered function, so that the body will tend to return to its original scheme if given the chance to do so.
5. Cells are selective at the time of accepting beams and vibrations and also at the time of rejecting any bemas and vibrations that they do not need. Should the cells suffer the lack of colour, that is another name for food, they then begin to depolarize and change their frequency and, consequently, their growth model, being even capable of performing an erroneous reproduction of the genetic code.
6. The application of an erroneous colour or food tends to change the frequency of the force of the electromagnetic field of the cell so that this force interacts with the wider magnetic field of the organ which does then affect the system that reacts upon the total force of the body field (typical chain reaction). This change leads to fatigue and a high level of fatigue is the cause of exhaustion and death.
7. Being pure vibration, colour is the rational type of therapy for health and illness, due to the fact that it has the correct form, in the appropriate place and moment in time.

It is not the aim of this document to develop at textbook level the chromotherapy technique, but simply to point out the documental backgrounds of apparatuses used to apply it, and in this sense we may well mention ES 9200803, which proposes a chromotherapy device for therapeutic purposes, although it deals with corporal aesthetics and, more specifically, with the removal of skin wrinkles and with skin tightening.

The device developed by the previously mentioned invention is based on the principle of cellular excitation using chromatic radiation obtained from a light emitting device conveyed using optical fibre in one or more conduits and fitted with on and off switching means at a given frequency to enable the light to produce an given effect upon the area of application by reason of its presence and the opposite effect upon its absence. It is made up through the functional association of a light emitting lamp, an electronic control circuit and a timer used to establish the operating times, joined to a remote operation pedal.

The beam generated by the light emitting lamp is taken through a filter until it reaches a quartz crystal applicator by way of optical fibre conduits acting as the transmission element.

As may be readily observed, it is a specific device that needs to be operated by a specialist and that is therefore fully removed from the purpose of the invention described below.

### DESCRIPTION OF THE INVENTION

The invention evidently originates from the chromatography technique but, as already mentioned in the opening statement, proposes a selective chromotherapy self application device that would enable the patient himself to use it following clear instructions supplied by the specialist, selecting the most appropriate time, both physical and mental, for the application of the treatment that has been specially prescribed for its specific requirements.

This approach makes it possible to free the patient from the schedules set out by treatment clinics where it is necessary to make appointments and waste time waiting, makes it less expensive to treat chronic diseases such as migraine or headaches, pre-menstruation pain, rheumatic complaints and any type of long or repetitive treatment not requiring a multiple treatment facility such as those normally available at surgeries, but a specific arrangement of coloured crystals enabling the emission of a given colour temperature and a vibration specific to the complaint to be treated.

With these specifications it is easy to custom make for each patient the self application device required for each case and to enable it to be immobilized on the area to be treated. Thus the coloured crystals capable of making light vibrate may form part of a mask, a chest covering piece, an apron or a foot covering template so that it may act by reason of added reflexology, lest we forget that this technique, together with acupuncture originate from the same school and are based on a common interrelation: the reactivation of the cells reestablishing their energy level.

Basically, the invention originates from a mineral and organic crystal that is cut in accordance with a previously established geometric pattern and dyed with a colour as per the criterion of the treating doctor as a function of the complaint to be treated. Thus, for instance, the yellow colour activates the lymphatic system, exerts a stimulating action and is therefore appropriate for the treatment of depressions but is counter-indicated for the treatment of neuralgias and palpitations, whereas the orange colour is appropriate of the treatment of asthma.

Considering all of the above, it is the case that, being within reach of duly qualified experts all of this knowledge, already amply reflected in scientific literature, up to now it has been necessary to undergo treatment exclusively at a surgery, which happens to be always tiring, expensive and hardly compatible with the schedules normally imposed by any type of working or personal activity, resulting in an ever increasing level of frustration of the patients and contributing to separate him from a rational use of the therapy, causing a lengthening or delay in respect of the achievement of the optimal treatment results.

Returning to the object of the invention, it is necessary to emphasize that, to enable the light to vibrate and to be able to modulate in amplitude and frequency, there must be natural quartz covering the coloured crystal, that is to say, quartz in which the impurities resulting from traces of other minerals are maintained, and to achieve this is the essence of the invention. The way in which this is achieved is so simple that it is precisely this the source of the essence and inventive activity that enable this proposal to deserve the privilege of becoming a patent specification.

The invention consists of arranging upon a coloured crystal a layer of mineral quartz milled to a variable grain size distribution ranging between dust and three millimetres, fixedly spread over the crystal using adhesive or arranged inside a chamber determined between two crystal films, one of them dyed and the other not, being the dyed film affected by transparent lines that allow the passage or geometrically controlled transparent beams.

The quarts granulates used in the second case may be partially fixed or fully free but, in respect of any way of putting the invention into practice, each such set is analyzed using a colour temperature meter and the wave length is measured using an oscilloscope to enable the determination of the appropriate characteristics.

Once the diagnosis is made and the proper mean is obtained, the selective self application set is then assembled upon its positioning support and, using a halogen light source with a power rating of, for instance, 50 to 75 W, the patient himself may then, following the prescribed therapy in respect of times and frequencies of application, follow treatments at his own convenience and at an affordable cost level, duly maintaining his medical consultation relation with his medical specialist who, may then, as a function of the results obtained, further adapt the treatment required to any progresses made.

As a complement to this document are attached drawings that depict examples of execution of the invention that merely pretend to illustrate its practical possibilities, without there being any limitations in respect of its execution or in the way in which the various crystals may be matched in a positioning support to form the self application device.

### DESCRIPTION OF THE DRAWINGS

In accordance with all of the above, figure one depicts a perspective view of a crystalline disc shaped element on which upper surface may be observed a heterogeneous granular dispersion or crushed mineral quartz crystals fixed using transparent adhesive in order to keep them immobile upon the coloured crystal on which they are set.

Figure two represents a self application device made up of three portions, between which there is a chamber inside which crushed mineral quartz may be arranged so that part of it may be fixed whereas the other part may be loose, so that the user himself may, within certain previously established parameters, modify its longitude, frequency and direction.

Figure three shows a chest covering piece, either opaque o transparent, where have been embedded crystals bearing shapes and colours chosen for the application of a given treatment to heal a specific complaint. Let us remember in this regard that the light shall vibrate within a colour temperature expressed in degrees Kelvin as per the colour of the mineral or organic crystal only in the presence of mineral quartz granulate bearing the typical impurities that confer it that property, as peculiar as basic in chromatography.

Figure four depicts a face mask with an opening for the mouth, the mask of reference is opaque in order to prevent sight fatigue and has been designed for therapeutical effects to treat neuralgias and sinusitis.

Figure five represents a foot template for the practice of chromotherapy together with reflexology, so that various techniques may work together towards the same healing objective.

### DESCRIPTION OF A PREFERRED EXECUTION EXAMPLE

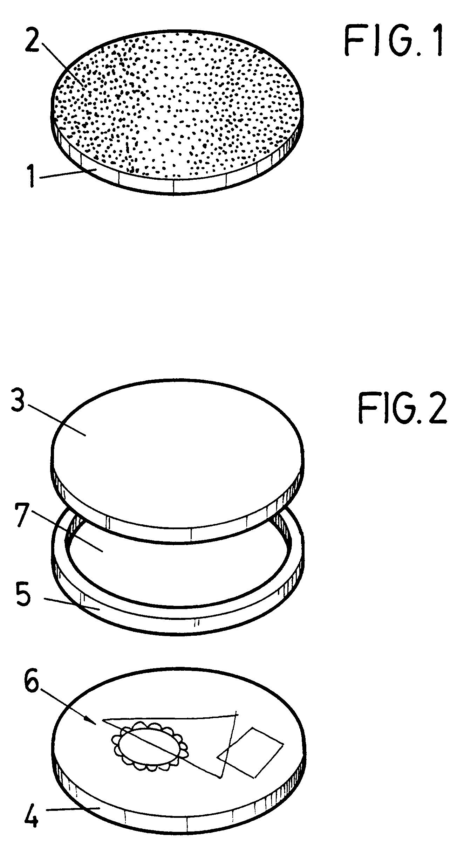

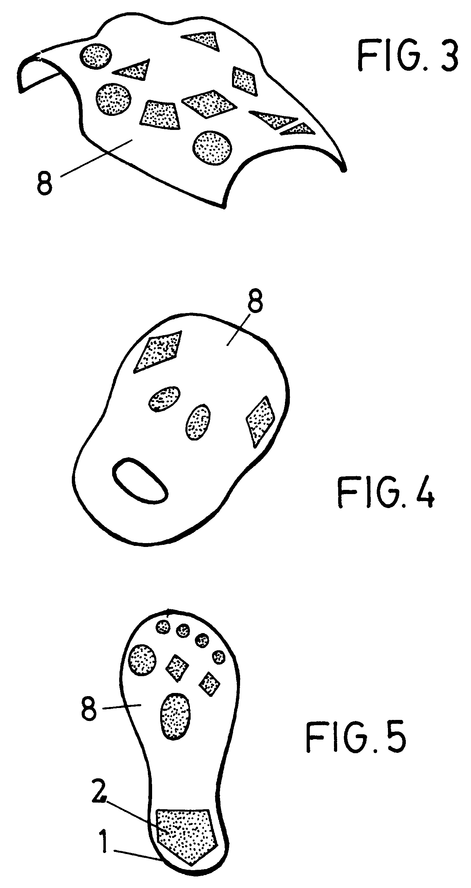

In accordance with the drawings, the fundamental portion incorporates a supporting device 1 of translucent nature based on mass dyed mineral or organic crystal acting as a support of one or more disperse layers of natural mineral quartz 2 crushed to a grain size distribution ranging from impalpable dust to three millimetres, being the crushed quarts fully or partially adhered to its supporting surface 1 or else arranged inside a chamber (7) formed between two transparent plates 3-4 arranged, for instance, on both sides of a ring 5, so as to form a chamber where the granulated natural mineral quartz 2 may be deposited, which mineral quartz has not been shown in figure two in order to simplify the drawing. Notwithstanding this, in this figure are shown on plate 4 geometrical drawings 6 that are etched separately of the colour in order to geometrically reorient the passage of the vibratory light so as to favour the treatment.

The shape of the coloured crystal base 1 is specific and is geometrically etched to form any kind of regular or irregular shape so that only the treating specialist may determine its size, colour and shape, so as to enable it to perform as per the relevant effect arising from the diagnosis. The purpose of the crushed natural quartz is to produce a given vibration that varies in amplitude and frequency in each case, to which end it is necessary to duly calibrate and position the crystal plus crushed quartz set in order to properly orientate the colorimetrically radiated vibration.

The possibility of integrating the crystals 1 on a setting capable of rotating around an axis, so that in certain cases it would be possible to periodically orientate the vibration, must not be neglected.

Another characteristic of the invention resides in the integration of one or more sets of crystal plus crushed natural quartz, using any of the various possibilities of execution, in a moulded piece that constitutes a template 8 for the proper arrangement of the crystals, so that they may then be directly positioned above the required area of operation of the body.

It is evident, upon examining the accompanying drawings and reading the above descriptions, that the shape of the template is specific in each case, and that the number of crystals and their colour may be combined as a function of the desired effects so that, for a person expert in this matter, it would not be necessary to supply any further explanations in order to enable that person to understand the scope of the invention and further enable that person to, based on the essence herein advanced, effect whichever number of practical variations he may be capable of implementing without straying from the fundament of the invention.

## Claims

1. Selective chromotherapy self application device, that being of the type based on the vibratory activation of a coloured light source whenever this light source passes through a natural quartz crystal containing traces of foreign matter and transparency imperfections, is characterized in that it incorporates a mass dyed supporting element 1 of a translucent nature based on mineral or organic crystal that acts as the supporting element of one or more disperse layers of natural mineral quartz 2, crushed down to a grain size distribution ranging from impalpable dust to three millimetres, being the crushed quartz fully adhered to its supporting element 1 or partially adhered and arranged within an enclosed chamber 7.

2. Selective chromotherapy self application device according to the prior claim, characterized in that the chamber 7 formed between two transparent plates 3-4 arranged on both sides of a ring 5 so as to form a chamber in which the crushed natural mineral quartz granules may be deposited, having provided on plate 4 geometric drawings 6 that are etched irrespectively of the colour in order to geometrically reorient the passage of the vibratory light whenever it passes through a line or combination of lines 5 that are colourless or opaque.

3. Selective chromotherapy self application device according to the prior claim, characterized in that the shape of the coloured crystal base 1 is specific and may be geometrically set out into any kind of regular or irregular shape, being the crystals 1 advantageously integrated on a mounting assembly capable or rotating around an axis so that in certain cases it may be feasible to periodically orientate the vibration.

4. Selective chromotherapy self application device according to the prior claims, characterized in that the crystal bases 1 plus the adhered quartz and/or a combination of plates 3-4 may be combined in respect of shape, colour and vibratory capacity into a moulded shape that constitutes a template 8 where the sets may be arranged, enabling their direct positioning upon the required body area.
